# FASCICULE DE BREVET EUROPEEN

(11) **EP 0 911 003 B1**
(45) Date de publication et mention de la délivrance du brevet: **23.06.2004**
(21) Numéro de dépôt: 98402179.0
(22) Date de dépôt: 03.09.1998
(51) Int. Cl.: A61F 11/08

(54) **Protection auditive contre les bruits élevés**
Gehörschutz gegen starkes Geräusch
Hearing protector against high noise

(30) Priorité: 18.09.1997 FR 9711623
(43) Date de publication de la demande: 28.04.1999
(73) Titulaire: I.S.L. Institut Franco-Allemand de Recherches de Saint-Louis, 68301 Saint-Louis Cedex (FR)
(72) Inventeur: Hamery, Pascal, 68100 Mulhouse (FR)
(74) Mandataire: Le Moenner, Annie

(56) Documents cités:
- CA-A- 1 041 439
- DE-C- 310 341
- FR-A- 2 676 642
- US-A- 2 465 606
- US-A- 3 736 929
- US-A- 5 483 027

## Description

La présente invention concerne une protection auditive contre des bruits élevés continus ou impulsionnels (jusqu'à 190 dB), pouvant fonctionner en mode d'atténuation sélective ou en mode d'atténuation maximale. Une protection auditive selon le préambule de la revendication 1 est connue du document US-A-2 465 606.

En mode d'atténuation sélective, l'atténuation des sons est faible pour une gamme de fréquences définie, et elle augmente pour les sons de fréquence supérieure à ceux de la gamme définie. L'atténuation sélective est particulièrement efficace pour les bruits les plus forts. Un exemple d'application d'une protection auditive en mode d'atténuation sélective est la transmission intelligible de la parole dans un environnement perturbé par des bruits impulsionnels tels que des bruits d'armes. Dans ce cas, la gamme de fréquences définie dans laquelle l'atténuation est faible est de 1000 à 3000 Hz.

En mode d'atténuation maximale, la protection auditive arrête tous les sons sur toute la gamme de fréquences, quelque soit leur intensité.

Selon le brevet FR 2 676 642 au nom de la demanderesse il existe une protection auditive peu encombrante en contact avec le conduit auditif, comprenant essentiellement un corps souple allongé, dans lequel sont placés des moyens d'atténuation sélective et des moyens d'atténuation maximale, et m obturateur d'un canal à commande manuelle permettant de commander le mode de fonctionnement en atténuation sélective ou maximale.

Toutefois, ce dispositif nécessite une manipulation délicate de l'utilisateur devant obturer le canal par lui-même. Cette manipulation peut être imparfaite et l'atténuation sélective ou maximale ainsi recherchée par obturation ne sera pas optimale.

Le but de l'invention est de proposer Lme protection auditive fiable qui ne présente pas les inconvénients du réglage par l'utilisateur et permette deux configurations d'atténuation de bruits de caractéristiques différentes.

L'invention a donc pour objet une protection auditive pour atténuer sélectivement ou non des bruits pouvant avoir une intensité jusqu'à 190 dB destinée à être insérée de manière étanche dans le conduit auditif, caractérisée en ce qu'elle est constituée d'un corps cylindrique souple présentant à chaque extrémité un embout, l'un des deux embouts comportant un canal débouchant d'une part au niveau du centre du corps considéré longitudinalement et d'autre part à l'une des extrémités de l'embout et contenant un filtre acoustique.

La protection auditive selon l'invention présente deux extrémités aptes l'une comme l'autre à être insérées dans le conduit auditif. Elle est dite "tête-bêche" ou à "double face". Contrairement à une protection auditive classique qui comporte en général une seule extrêmité apte à être insérée dans le conduit auditif, l'autre extrémité permettant de tenir la protection auditive afin de la positionner dans le conduit auditif, la présente invention comprend deux extrémités, identiques ou non, pouvant l'une ou l'autre être insérées dans le conduit auditif, permettant ainsi de choisir parmi deux modes de fonctionnement en atténuation identiques ou non.

L'intérêt d'un tel dispositif réside dans le fait qu'il possède, au sein d'une même protection auditive, deux configurations pouvant présenter des caractéristiques d'atténuation différentes, toutes deux obtenues par simple inversion du sens d'insertion de la protection auditive ou bouchon d'oreille.

Dans un mode de réalisation préférentiel, les deux embouts sont deux pièces distinctes reliées par une pièce de jonction interne.

La pièce de jonction peut être un cylindre unique, percé d'un alésage renfermant un filtre acoustique et formant un angle droit débouchant d'une part par une première extrémité dans le canal, et d'autre part par une deuxième extrêmité au niveau du centre de la pièce de jonction.

La pièce de jonction peut être un cylindre unique comportant un alésage débouchant en trois endroits : au niveau du centre de la pièce de jonction, et à chaque extrêmité de la pièce de jonction, les parties d'alésage débouchant aux extrémités renfermant chacune un filtre acoustique identique ou différent.

La pièce de jonction peut aussi être constituée de trois parties cylindriques , la partie centrale étant percée d'un alésage en son centre et ayant un diamètre légèrement supérieur à celui des deux autres parties, qui ont un diamètre sensiblement égal et légèrement supérieur à celui du canal, et dont l'une au moins est percée d'un alésage en son centre, qui renferme un filtre acoustique et qui communique avec l'alésage de la partie centrale. Lorsque les deux parties renferment chacune un filtre acoustique, ce dernier peut être identique ou différent.

Dans un autre mode de réalisation, la pièce de jonction peut présenter des aspérités pour le maintien des embouts.

Dans un autre mode de réalisation, elle peut avoir des extrêmités coniques.

Préférentiellement, les deux embouts de la protection auditive sont munis d'une face sensiblement hémisphérique dont le côté le moins large est destiné à être inséré en premier dans le conduit auditif.

Avantageusement le corps de la protection auditive est pourvu d'ailettes annulaires souples de diamètre croissant de l'intérieur vers l'extérieur du conduit auditif pour le calage dans le conduit auditif.

La protection auditive permet par le choix du filtre acoustique un filtrage non linéaire des sons.

D'autres caractéristiques de la présente invention apparaitront à la lecture de la description suivante d'un mode de réalisation préférentiel, donné à titre illustratif et non limitatif, et des figures annexées parmi lesquelles :
- la figure 1 représente en coupe longitudinale un premier mode de réalisation d'une protection auditive "double face" selon l'invention;
- la figure 2 représente en coupe longitudinale un deuxième mode de réalisation d'une protection auditive "double face" selon l'invention;
- la figure 3 représente en coupe longitudinale un troisième mode de réalisation d'une protection auditive "double face" selon l'invention;
- la figure 4 représente en coupe longitudinale des variantes de la pièce d'assemblage des deux faces de la protection auditive selon l'invention;
- la fugure 5 représente en perspective un mode de réalisation de la pièce d'assemblage des deux faces de la protection auditive selon l'invention;
- la figure 6 représente en coupe longitudinale un quatrième mode de réalisation d'une protection auditive selon l'invention.

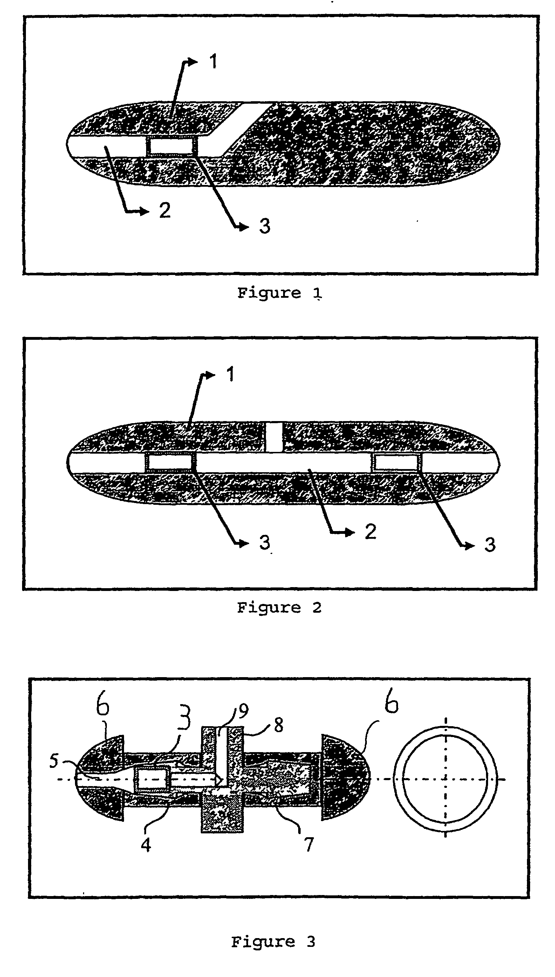

La figure 1 représente une configuration d'une protection auditive selon l'invention comportant un corps 1, qui est une pièce moulée s'adaptant au conduit auditif, percée d'un canal 2 partant de l'une des deux extrémités et débouchant au niveau du centre du corps 1. Le canal 2 renferme un filtre acoustique 3 permettant par exemple un filtrage sélectif et non linéaire des son. L'autre extrémité n'est pas perforée et permet une atténuation maximale quelle que soit la fréquence et quel que soit le niveau d'amplitude du son.

Le corps 1 a une longueur de 2 à 4 cm et est constitué d'une matière souple.

La figure 2 représente une configuration d'une protection auditive selon l'invention, dans laquelle le corps 1 est percé d'un canal 2 débouchant à chaque extrémité et au centre du corps 1, et contenant deux filtres acoustiques 3 vers chaque extrêmité. Les deux filtres peuvent être identiques ou non.

La figure 3 représente un mode de réalisation d'une protection auditive selon l'invention constitué de trois parties : deux embouts cylindriques creux 4 et 7, munis chacun d'une face sensiblement hémisphérique 6, dont le côté le moins large est destiné à être placé en premier dans le conduit auditif, et une troisième pièce interne de jonction 8 de ces deux embouts. La face sensiblement hémisphérique assure l'étanchéité entre la protection auditive et le conduit auditif.

L'un au moins des deux embouts est percé d'un canal 5 en son centre, dans lequel est insérée l'une des deux extrêmités de la pièce de jonction 8 qui renferme un filtre acoustique 3 permettant par exemple un filtrage sélectif et non linéaire des sons. Le deuxième embout peut ne pas être perforé, et dans ce cas il pennet une atténuation maximale quelle que soit la fréquence et quel que soit le niveau d'amplitude du son. Les deux embouts 4 et 7 sont deux pièces distinctes qui s'emboitent l'une sur l'autre et sont assemblées par la troisième pièce 8 permettant de les maintenir solidairement. Cette pièce d'assemblage 8 est percée d'un alésage 9 renfermant un filtre acoustique et présentant un angle droit débouchant d'une part par une première extrêmité dans le canal 5, permettant ainsi l'utilisation du filtre 3, et d'autre part par une deuxième extrémité au niveau du centre de la pièce 8.

Dans un autre mode de réalisation, la pièce 8 peut aussi comporter un alésage débouchant en trois endroits : au niveau du centre de la pièce 8, et à chaque extrêmité de la pièce 8, les parties d'alésage débouchant aux extrêmités renfermant chacune un filtre acoustique identique ou non.

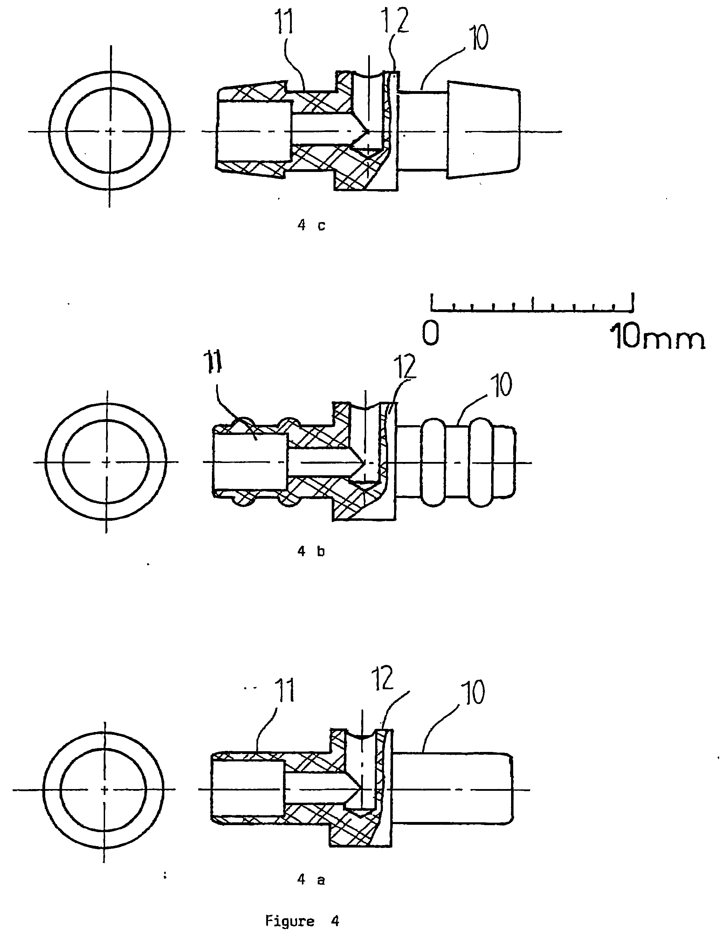

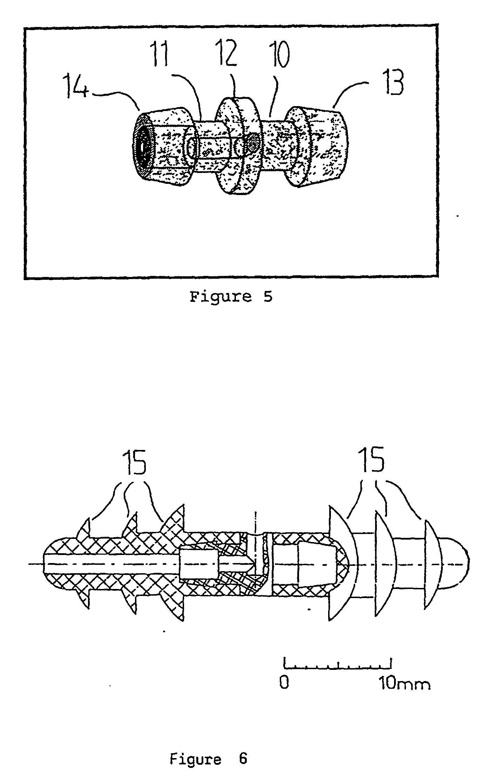

La pièce de jonction 8 peut être constituée simplement par un cylindre unique ou être constituée de trois parties cylindriques 10, 11, 12 comme sur la figure 4a. La partie centrale 12 est percée d'un alésage en son centre et a un diamètre légèrement supérieur à celui des deux autres parties 10 et 11, qui ont un diamètre sensiblement égal et légèrement supérieur à celui du canal 5 pour permettre un bon maintien de l'ensemble. L'une au moins des deux parties 10 et 11 est percée d'un alésage qui renferme un filtre acoustique et communique avec l'alésage de la partie 12, comme cela est indiqué sur la figure 5.

La pièce de jonction 8 peut présenter des aspérités comme sur la figure 4b ou bien avoir des extrêmités 13 et 14 de forme conique comme sur les figures 4c et 5. Dans le cas du mode de réalisation en trois parties, les parties 10 et 11 sont porteuses des aspérités et des extrêmités coniques.

L'un des deux embouts comporte une perforation qui vient lors de l'assemblage avec la pièce de jonction 8 en coincidence avec celle de l'extrêmité centrale du canal 9 située sur la partie 12.

La figure 6 représente une vue en coupe longitudinale d'une configuration de protection auditive selon l'invention, pourvue sur les embouts d'ailettes annulaires souples 15 pour assurer le calage de la protection auditive dans les parois du conduit auditif. Ces ailettes peuvent être de diamètre croissant de l'intérieur vers l'extérieur du conduit auditif.

La protection auditive selon l'invention est particulièrement utile pour les équipages d'avions ou de véhicules militaires en exercice, qui sont exposés à des bruits de moteurs et d'armes très élevés.

La protection auditive selon l'invention peut aussi être efficacement utilisée par tout personnel exposé à des bruits de forte intensité dans leur environnement de travail : chantiers de construction, carrières, etc.

## Revendications

1. Protection auditive pour atténuer sélectivement ou non des bruits pouvant avoir une intensité jusqu'à 190 dB destinée à être insérée de manière étanche dans le conduit auditif, ladite protection auditive étant constituée d'un corps (1) cylindrique souple présentant à chaque extrémité un embout, **caractérisée en ce que** l'un des deux embouts comporte un canal (2,5,9) débouchant d'une part au niveau du centre du corps (1) considéré longitudinalement et d'autre part à l'une des extrémités de l'embout et contenant un filtre acoustique (3).

2. Protection auditive selon la revendication 1, **caractérisé en ce que** chaque embout comporte un canal (2,5,9) débouchant d'une part au niveau du centre du corps (1) considéré longitudinalement et d'autre part à l'une des extrémités de l'embout et contenant un filtre acoustique (3)identique ou non.

3. Protection auditive selon l'une quelconque des revendications 1 et 2, **caractérisée en ce que** les deux embouts sont deux pièces distinctes reliées par une pièce de jonction interne (8).

4. Protection auditive selon les revendications 1 et 3, **caractérisée en ce que** la pièce de jonction (8) est un cylindre unique, percé d'un alésage (9) renfermant un filtre acoustique et formant un angle droit débouchant d'une part par une première extrémité dans le canal (5), et d'autre part par une deuxième extrémité au niveau du centre de la pièce de jonction (8).

5. Protection auditive selon les revendications 2 et 3, **caractérisée en ce que** la pièce de jonction (8) est un cylindre unique comportant un alésage débouchant en trois endroits : au niveau du centre de la pièce de jonction (8), et à chaque extrémité de la pièce de jonction (8), les parties d'alésage débouchant aux extrémités renfermant chacune un filtre acoustique identique ou non.

6. Protection auditive selon les revendications 1 et 3, **caractérisée en ce que** la pièce de jonction (8) est constituée de trois parties cylindriques (10, 11, 12), la partie centrale 12 étant percée d'un alésage en son centre et ayant un diamètre légèrement supérieur à celui des deux autres parties (10 et 11), qui ont un diamètre sensiblement égal et légèrement supérieur à celui du canal (5), et dont l'une est percée d'un alésage en son centre, qui renferme un filtre acoustique et qui communique avec l'alésage de la partie centrale (12).

7. Protection auditive selon les revendications 2 et 3, **caractérisée en ce que** la pièce de jonction (8) est constituée de trois parties cylindriques (10, 11, 12), la partie centrale (12) étant percée d'un alésage en son centre et ayant un diamètre légèrement supérieur à celui des deux autres parties (10 et 11), qui ont un diamètre sensiblement égal et légèrement supérieur à celui du canal (5), et qui sont toutes deux percées d'un alésage en son centre, qui communique avec l'alésage de la partie centrale (12) et qui renferme un filtre acoustique identique ou non.

8. Protection auditive selon l'une des revendications 3 à 7, **caractérisée en ce que** la pièce de jonction (8) présente des aspérités pour le maintien des embouts (4,7).

9. Protection auditive selon l'une des revendications 3 à 7, **caractérisée en ce que** la pièce de jonction (8) a des extrémités coniques (13,14).

10. Protection auditive selon l'une des revendications 1 à 9, **caractérisée en ce que** les deux embouts de la protection auditive sont munis d'une face sensiblement hémisphérique (6) dont le côté le moins large est destiné à être inséré en premier dans le conduit auditif.

11. Protection auditive selon l'une des revendications 1 à 10, **caractérisée en ce que** le corps (1) est pourvu d'ailettes annulaires souples (15) de diamètre croissant de l'intérieur vers l'extérieur du conduit auditif pour le calage dans le conduit auditif.

12. Protection auditive selon l'une des revendications 1 à 11, **caractérisée en ce que** le filtre acoustique permet un filtrage non linéaire des sons.

## Patentansprüche

1. Hörschutz zum selektiven oder nicht selektiven Unterdrücken von Geräuschen, die eine Stärke bis zu 190 dB haben können, der zum Einführen in den Gehörgang auf dichte Weise bestimmt ist, wobei der Hörschutz aus einem zylindrischen, elastischen Körper (1) besteht, der an jedem Ende einen Ansatz aufweist, **dadurch gekennzeichnet, daß** einer der beiden Ansätze einen Kanal (2, 5, 9) aufweist, der einerseits auf Höhe des Zentrums des Körpers (1), betrachtet in Längsrichtung, und andererseits an einem Enden des Ansatzes mündet und ein akustisches Filter (3) enthält.

2. Hörschutz nach Anspruch 1, **dadurch gekennzeichnet, daß** jeder der beiden Ansätze einen Kanal (2, 5, 9) aufweist, der einerseits auf Höhe des Zentrums des Körpers (1), betrachtet in Längsrichtung, und andererseits an einem der Enden des Ansatzes mündet und ein identisches oder nicht identisches akustisches Filter (3) enthält.

3. Hörschutz nach einem der Ansprüche 1 und 2, **dadurch gekennzeichnet, daß** die beiden Ansätze zwei getrennte Teile sind, die durch ein internes Verbindungsteil (8) verbunden sind.

4. Hörschutz nach den Ansprüchen 1 und 3, **dadurch gekennzeichnet, daß** das Verbindungsteil (8) ein einziger Zylinder ist, der von einer Bohrung (9) durchsetzt ist, die ein akustisches Filter umschließt und einen rechten Winkel bildet, der einerseits mit einem Ende in dem Kanal (5) und andererseits mit einem zweiten Ende auf Höhe des Zentrums des Verbindungsteils (8) mündet.

5. Hörschutz nach den Ansprüchen 2 und 3, **dadurch gekennzeichnet, daß** das Verbindungsteil (8) ein einziger Zylinder ist, der eine Bohrung aufweist, die an drei Stellen mündet: auf Höhe des Zentrums des Verbindungsteils (8) und an jedem Ende des Verbindungsteils (8), wobei die an den Enden mündenden Bohrungsteile jeweils ein akustisches Filter umschließen, das identisch ist oder nicht.

6. Hörschutz nach den Ansprüchen 1 und 3, **dadurch gekennzeichnet, daß** das Verbindungsteil (8) aus drei zylindrischen Teilen (10, 11, 12) gebildet ist, wobei der zentrale Teil (12) von einer Bohrung in seinem Zentrum durchsetzt ist und einen etwas größeren Durchmesser als die zwei anderen Teile (10 und 11) hat, die einen Durchmesser haben, der im wesentlichen gleich oder etwas größer als derjenige des Kanals (5) ist, und von denen einer von einer Bohrung in seinem Zentrum durchsetzt ist, die ein akustisches Filter umschließt und mit der Bohrung des zentralen Teils (12) in Verbindung steht.

7. Hörschutz nach den Ansprüchen 2 und 3, **dadurch gekennzeichnet, daß** das Verbindungsteil (8) aus drei zylindrischen Teilen (10, 11, 12) gebildet ist, wobei der zentrale Teil (12) von einer Bohrung in seinem Zentrum durchsetzt ist und einen etwas größeren Durchmesser als die zwei anderen Teile (10 und 11) hat, die einen Durchmesser haben, der im wesentlichen gleich oder etwas größer als derjenige des Kanals (5) ist, und die alle beide von einer Bohrung in ihrem Zentrum durchsetzt sind, die mit der Bohrung des zentralen Teils (12) in Verbindung steht und ein identisches oder nicht identisches akustisches Filter umschließt.

8. Hörschutz nach einem der Ansprüche 3 bis 7, **dadurch gekennzeichnet, daß** das Verbindungsteil (8) Rauheiten zum Halt der Ansätze (4, 7) aufweist.

9. Hörschutz nach einem der Ansprüche 3 bis 7, **dadurch gekennzeichnet, daß** das Verbindungsteil (8) konische Enden (13, 14) hat.

10. Hörschutz nach einem der Ansprüche 1 bis 9, **dadurch gekennzeichnet, daß** die zwei Ansätze des Hörschutzes mit einer im wesentlichen halbkugelförmigen Fläche (6) ausgestattet sind, deren weniger breite Seite dazu bestimmt ist, als erste in den Gehörgang eingeführt zu werden.

11. Hörschutz nach einem der Ansprüche 1 bis 10, **dadurch gekennzeichnet, daß** der Körper (1) mit elastischen, ringförmigen Flügeln (15) mit vom Inneren zum Äußeren des Gehörgangs wachsendem Durchmesser zum Verkeilen in dem Gehörgang versehen ist.

12. Hörschutz nach einem der Ansprüche 1 bis 11, **dadurch gekennzeichnet, daß** das akustische Filter eine nichtlineare Filterung der Töne ermöglicht.

## Claims

1. Auditory protection device for attenuating, selectively or non-selectively, noises capable of having an intensity of up to 190 dB, which device is intended for insertion in an impervious manner in the auditory canal, the said auditory protection device being made up of a flexible cylindrical body (1) having a tip at each end, **characterised in that** one of the two tips comprises a duct (2, 5, 9) which terminates, on the one hand, at the level of the centre of the body (1), considered longitudinally, and on the other, at one of the ends of the tip, and which contains an acoustic filter (3).

2. Auditory protection device according to claim 1, **characterised in that** each tip comprises a duct (2, 5, 9) which terminates, on the one hand, at the level of the centre of the body (1), considered longitudinally, and on the other, at one of the ends of the tip, and which contains an acoustic filter (3), said filters being identical or non-identical.

3. Auditory protection device according to any one of claims 1 and 2, **characterised in that** the two tips are two distinct pieces connected by an internal junction-piece (8).

4. Auditory protection device according to claims 1 and 3, **characterised in that** the junction-piece (8) is a single cylinder perforated by a bore (9) enclosing an acoustic filter and forming a right angle, which terminates, on the one hand, in the duct (5) via a first end and, on the other, at the level of the centre of the junction-piece (8) via a second end.

5. Auditory protection device according to claims 2 and 3, **characterised in that** the junction-piece (8) is a single cylinder comprising a bore that terminates in three places: at the level of the centre of the junction-piece (8) and at each end of said junction-piece (8), those parts of the bore which terminate at the ends each enclosing an acoustic filter, said filters being identical or non-identical.

6. Auditory protection device according to claims 1 and 3, **characterised in that** the junction-piece (8) is made up of three cylindrical parts (10, 11, 12), the central part 12 being perforated by a bore in its centre and having a diameter which is slightly larger than that of the other two parts (10 and 11) which have a diameter which is substantially equal and slightly larger than that of the duct (5) and of which one is perforated by a bore in its centre, which encloses an acoustic filter and which communicates with the bore in the central part (12).

7. Auditory protection device according to claims 2 and 3, **characterised in that** the junction-piece (8) is made up of three cylindrical parts (10, 11, 12), the central part (12) being perforated by a bore in its centre and having a diameter which is slightly larger than that of the other two parts (10 and 11) which have a diameter which is substantially equal and slightly larger than that of the duct (5) and which are both perforated by a bore in their centre, which communicates with the bore in the central part (12) and which encloses an acoustic filter, said filters being identical or non-identical.

8. Auditory protection device according to one of claims 3 to 7, **characterised in that** the junction-piece (8) has rough portions for holding the tips (4, 7) in position.

9. Auditory protection device according to one of claims 3 to 7, **characterised in that** the junction-piece (8) has conical ends (13, 14).

10. Auditory protection device according to one of claims 1 to 9, **characterised in that** the two tips of the auditory protection device are equipped with a substantially hemispherical face (6), the least wide side of which is intended to be inserted first in the auditory canal.

11. Auditory protection device according to one of claims 1 to 10, **characterised in that** the body (1) is provided with flexible annular fins (15) having a diameter that increases from the inside towards the outside of the auditory canal for the purpose of being wedged in the said auditory canal.

12. Auditory protection device according to one of claims 1 to 11, **characterised in that** the acoustic filter permits non-linear filtering of sounds.
